# EUROPEAN PATENT APPLICATION

(11) **EP 1 666 168 A1**
(43) Date of publication of application: **07.06.2006**
(21) Application number: 04788181.8
(22) Date of filing: 27.09.2004
(51) Int. Cl.: B08B 9/02, A61B 17/00

(54) **TOOL FOR REMOVING FOREIGN OBJECT IN PIPE LINE**

(30) Priority: 26.09.2003 JP 2003335749
(71) Applicant: Nihon University, Tokyo 102-8275 (JP); IR Medical Laboratory Co., Ltd., Koriyama-shi, Fukushima 963-8871 (JP)
(72) Inventor: Omata, Sadao, Chiyoda-ku, Tokyo 1028275 (JP); Hyogo, Toshio, Sapporo-shi, Hokkaido 0640912 (JP); Kawabe, Daisuke c/o IR Medical Laboratory co.,Ltd., Koriyama-shi, Fukushima 9638871 (JP); Murayama, Kazuya c/o IR Medical Laboratory co.,Ltd, Koriyama-shi, Fukushima 9638871 (JP)
(74) Representative: Henseler, Daniela
(86) International application number: PCT/JP2004/014085
(87) International publication number: WO 2005/030408

(57) **Abstract**

To provide an intraductal foreign body removal instrument which can remove fibrous foreign bodies 80 out of a duct 90 without damaging inner walls of the duct 90 using a simple mechanism. The instrument has a flexible insertion tube 20 inserted in the duct 90, a wire 30 made of flexible wire material and inserted in the insertion tube 20; and a rotating device 40 which rotates the wire 30 in the insertion tube 20. Furthermore, the instrument 10 has a flexible guide tube 25 which is inserted in the duct 90 and into which the insertion tube 20 is inserted loosely.

## Description

### Technical Field

The present invention relates to an intraductal foreign body removal instrument used to remove fibrous foreign bodies out of ducts. More particularly, it relates to an intraductal foreign body removal instrument which can be suitably used as a catheter to remove fibrous occluding objects and the like out of intravital ducts such as blood vessels.

### Background Art

Conventionally, intraductal foreign body removal instruments have been developed to remove fibrous foreign bodies clogging constrictions and the like in ducts through which a fluid passes.
A technique described in JP10-175705A discloses an intraductal foreign body removal instrument, comprising a gripping mechanism which is attached to a main unit (foreign body removal instrument) to grip, for example, foreign bodies in a duct using claws, wherein a tip of a pull-up wire is attached to the main unit, and the other end of the wire is attached to a wire winder installed outside the duct.
According to this technique, the main unit is fed into a duct until it gets close to a constriction caused by foreign bodies. Then the gripping mechanism is brought close to the foreign bodies and the claws are closed to grip the foreign bodies. Then the pull-up wire is wound by the winder, taking the main unit out of the duct, and thereby removing the foreign bodies.

### Disclosure of the Invention

However, an intraductal foreign body removal instrument comprising a gripping mechanism, as is the case with the technique disclosed in JP10-175705A, involves feeding the gripping mechanism itself composed of a large number of parts into a duct, which increases resistance in the duct. Consequently, for example, elbows or similar bends increase resistance, causing a problem of limited feed. Also, to position the gripping mechanism in the duct and remove (grip) foreign bodies, it is necessary to control relative distance from the foreign bodies by subtly adjusting an actuator. This presents a problem of increased time required to remove the foreign bodies.

Furthermore, under circumstances where a duct has a small diameter allowing a limited work area, limited space may be available for use to open and close the gripping mechanism. Also, there may be cases in which the gripping mechanism itself cannot be fed to a constriction in the duct due to its size.
Possible circumstances where a duct has a small diameter allowing a limited work area include an intravital duct from which foreign bodies are removed. When removing foreign bodies out of an intravital duct, which is small in diameter and twists turns intricately, without damaging body tissues, the above problems become more prominent.
A catheter, for example, is used to remove foreign bodies out of intravital ducts. Although various catheters have been developed in pursuit of less invasive therapies for various diseases, they leave room for further studies.

For example, when removing a fibrous thrombus from a constricted blood vessel, there is a problem in that after a balloon catheter for percutaneous transluminal coronary angioplasty and the like is used mainly to dilate the constricted blood vessel, a retrieval catheter must be inserted separately to retrieve (remove) fine fragments of the thrombus produced when the constriction is dilated by the balloon.
The present invention has been made in view of the above problems and has an object to provide an intraductal foreign body removal instrument which can remove fibrous foreign bodies clogging elbows, narrowed parts, or other constrictions of pipes or similar ducts without requiring a large, complex equipment configuration. More particularly, it has an object to provide an intraductal foreignbody removal instrument which can be suitably used as a catheter to be inserted in intravital ducts such as blood vessels to remove fibrous occluding obj ects out of the duct.

To achieve the above object, the present invention as set forth in claim 1 provides an intraductal foreign body removal instrument which removes fibrous foreign bodies out of a duct, characterized by comprising: a flexible insertion tube inserted in the duct; a wire made of flexible wire material and inserted in the insertion tube; and a rotating device which rotates the wire in the insertion tube.
The invention set forth in claim 1 allows the insertion tube and the wire in the insertion tube to be reduced in diameter, making it possible to insert them easily in a duct. Besides, that side (hereinafter referred to as a free end side) of the intraductal foreign body removal instrument which is inserted in a duct is not equipped with any special mechanism (such as the gripping mechanism of the conventional example). That is, when removing fibrous foreign bodies out of a duct using the intraductal foreign body removal instrument, the free end side is fed into the duct, but it does not encounter much resistance because it is only the insertion tube (and the wire in the insertion tube). Consequently, even elbows and similar bends, for example, do not offer high resistance. Thus, the instrument can be used even in small-diameter ducts. Also, feed limits of the intraductal foreign body removal instrument can be increased.

When the free end side of the intraductal foreign body removal instrument is inserted to a desired position, fibrous foreign bodies can be tangled around the free end side of the intraductal foreign body removal instrument by rotating the wire by the rotating device. After the fibrous foreign bodies are tangled, they can be removed as the intraductal foreign body removal instrument is retrieved from the duct through insertion position.
The mechanism by which fibrous foreign bodies can be tangled around the free end side of the intraductal foreign body removal instrument through rotation of the wire is not completely clear, but is presumed to be as follows.

When the wire rotates, its whirling causes the insertion tube to vibrate. The vibration lowers static pressure near the free end side of the intraductal foreign body removal instrument. Consequently, fluid convection(eddy) toward the intraductal foreign body removal instrument occurs in the duct. The vibration and convection produce a force which moves the fibrous foreign bodies toward the free end side of the intraductal foreign body removal instrument, causing them to get tangled around the free end side.
In this way, the intraductal foreign body removal instrument according to the present invention can remove fibrous foreign bodies clogging elbows, narrowed parts, or other constrictions of pipes or similar ducts without requiring a large, complex equipment configuration.
Claim 2 of the present invention sets forth the intraductal foreign body removal instrument according to claim 1, characterized in that the intraductal foreign body removal instrument is a catheter inserted into an intravital duct.

The invention set forth in claim 2 allows a small-diameter catheter with a simple mechanism to be constructed, making it possible to remove fibrous foreign bodies out of intravital ducts without damaging inner walls of the ducts. Thus, the intraductal foreign body removal instrument is less invasive to the patients.
Claim 3 of the present invention sets forth the intraductal foreign body removal instrument according to claim 1 or 2, characterizedby further comprising a flexible guide tube which is inserted in the duct and into which the insertion tube is inserted loosely.

The invention set forth in claim 3, according to which the insertion tube is loosely inserted into the guide tube, allows the guide tube and insertion tube to move relative to each other in the longitudinal direction. If the clearance between the guide tube and insertion tube is designed to accommodate fibrous foreign bodies tangled around the free end side of the insertion tube, when withdrawing the intraductal foreign body removal instrument from the duct through insertion position, the wire inside insertion tube and insertion tube can be retrieved together with the fibrous foreign bodies after being stored in the guide tube. This suitably prevents the fibrous foreign bodies from falling off the free end side of the insertion tube as well as prevents them from damaging inner walls of the duct during retrieval. Also, where the insertion tube is covered by the guide tube, the vibration of the insertion tube can be reduced by the guide tube.

### Brief Description of the Drawings

Figure 1 is a schematic block diagram showing an embodiment of an intraductal foreign body removal instrument according to the present invention;
Figures 2A to 2D are explanatory diagrams illustrating a process of removing fibrous foreign bodies out of a duct using the intraductal foreign body removal instrument according to the present invention;
Figures 3A to 3C are explanatory diagrams illustrating a variation of the intraductal foreign body removal instrument according to the present invention;
Figure 4 is an explanatory diagram illustrating a variation of the intraductal foreign body removal instrument according to the present invention; and
Figures 5A and 5B are explanatory diagrams illustrating an example of (experiment with) the intraductal foreign body removal instrument according to the present invention, where Figure 5A is a schematic block diagram showing an overall view of an experimental arrangement and Figure 5B is an enlarged view of part A in Figure 5A.

### Description of Symbols

- 10: Intraductal foreign body removal instrument
- 20: Insertion tube
- 25: Guide tube
- 30: Wire
- 40: Rotating device
- 50: Blood vessel model (experimental arrangement)
- 60: Circulation unit
- 62: Narrowed part
- 64: Elbow
- 66: Connecting pipe
- 70: Tank
- 80: (Fibrous) foreign bodies
- 90: Duct

### Best Mode for Carrying Out the Invention

An embodiment of an intraductal foreign body removal instrument according to the present invention will be described below by referring to drawings as required.
In this embodiment, the intraductal foreign body removal instrument according to the present invention is configured to be suitably used as a catheter inserted in an intravital duct to remove fibrous occluding objects out of the duct under circumstances, such as in a living body, where the duct is narrow and tortuous allowing a limited work area. Incidentally, examples of fibrous occluding objects in intravital ducts include thrombus produced in blood vessels.

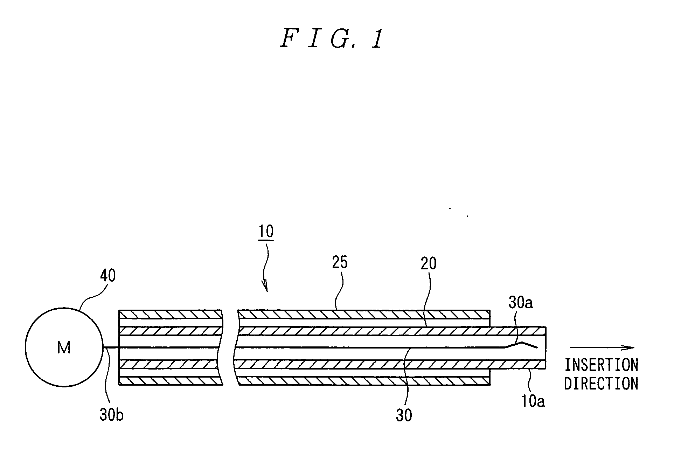

Figure 1 is a schematic block diagram of the intraductal foreign body removal instrument 10 according to the present invention. Incidentally, the figure shows a sectional view of an insertion tube and guide tube along the longitudinal axis.
As shown in the figure, the intraductal foreign body removal instrument 10 comprises a flexible insertion tube 20 inserted in a duct, a wire 30 made of flexible wire material and passed through the insertion tube 20, and a rotating device 40 which rotates the wire 30 in the insertion tube 20.

Furthermore, the intraductal foreign body removal instrument 10 comprises a guide tube 25 inserted into the duct together with the insertion tube 20 containing the wire 30. Thus, the insertion tube 20 containing the wire 30 is passed through the guide tube 25.
Specifically, the insertion tube 20 consists of annular cross sections extending continuously in the longitudinal direction and is open at both ends. It is a thin-walled, 0.5-mm diameter (outside diameter) tube formed of highly transparent fluororesin (PFA) with excellent heat and chemical resistance.

The wire 30 is made of wire material of a nickel-titanium alloy and is 0.2 mm in diameter. As shown in Figure 1, a roughly dogleg bend 30a is formed on that side (hereinafter referred to as a free end side) 10a of the wire 30 which is inserted into the target duct as the intraductal foreign body removal instrument 10 is inserted.
Any actuator which can output a rotary motion can be suitably used as the rotating device 40. According to this embodiment, that end 30b of the wire 30 which is opposite to the bend 30a is connected to an output shaft of the rotating device 40 via a connection joint (not shown). Consequently the rotating device 40 can rotate the wire 30 in the insertion tube 20.

As in the case of the insertion tube 20, the guide tube 25 consists of annular cross sections extending continuously in the longitudinal direction and is open at both ends. It is a thin-walled tube formed of highly transparent fluororesin (PFA) with excellent heat and chemical resistance. The inside diameter of the guide tube 25 is larger than the outside diameter of the insertion tube 20 so that the insertion tube 20 can be inserted loosely into the guide tube 25 with a predetermined clearance.
Next, description will be given of how to use the intraductal foreign body removal instrument 10 configured as described above as well as its operation and effect.

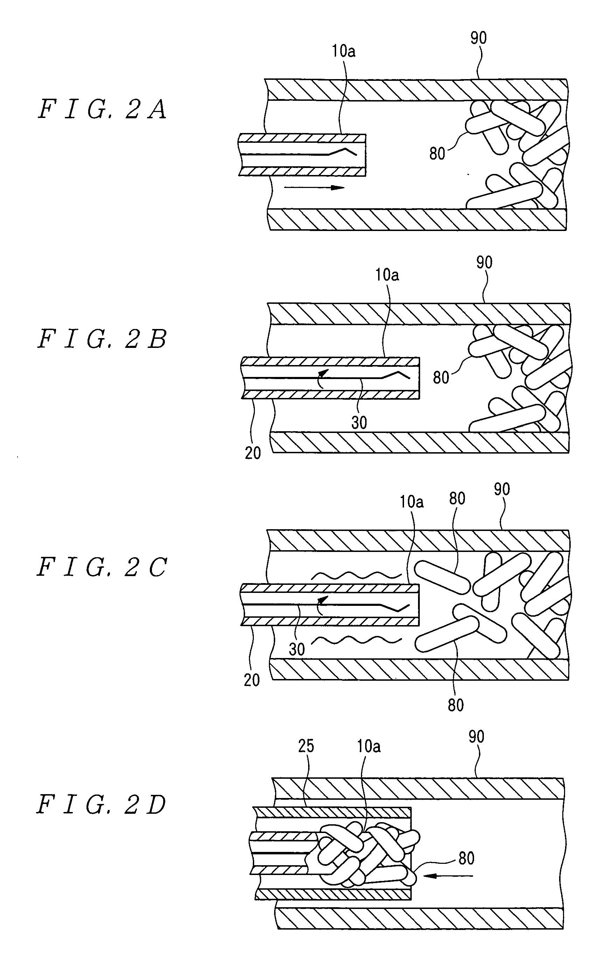

Figures 2A to 2D are explanatory diagrams illustrating a process of removing fibrous foreign bodies out of a duct using the intraductal foreign body removal instrument 10. Figures 2A to 2D show sectional views of a duct 90 and the intraductal foreign body removal instrument 10 along the longitudinal axis.
As shown in Figure 2A, first the free end side 10a of the intraductal foreign body removal instrument 10 is inserted through an opening of the duct 90 to or near a constriction caused by fibrous foreign bodies 80 in the duct 90.

At this time, the guide tube 25 is inserted together, but made to wait a little behind the free end side 10a inserted to or near the constriction caused by the fibrous foreign bodies 80. Thus, the guide tube 25 is not shown in Figure 2A (the same is true in Figures 2B and 2C). Incidentally, in Figure 2A, the insertion of the free end side 10a into the duct 90 is conceptually indicated by an arrow near the free end side 10a.
Next, as shown in Figure 2B, when the free end side 10a of the intraductal foreign body removal instrument 10 is inserted to a predetermined position (near the constriction in the figure) in the duct 90, the wire 30 is rotated in the insertion tube 20 by the rotating device 40 (not shown in Figures 2A to 2D) . Incidentally, the rotation of the wire 30 in the insertion tube 20 is conceptually indicated by an arrow around the wire 30 (the same is true hereinafter).

As shown in Figure 2C, when the wire 30 rotates in the insertion tube 20, its whirling causes the insertion tube 20 to vibrate. Consequently, the fibrous foreign bodies 80 are drawn to the free end side 10a from the constriction. Here, only the wire 30 is rotated directly by driving force of the rotating device 40, and the insertion tube 20 around the wire 30 is not rotated directly by the driving force of the rotating device 40. Consequently, inner walls of the intravital duct 90 are hardly damaged. Incidentally, vibration of the insertion tube 20 caused by the whirling of the wire 30 rotating in the insertion tube 20 is conceptually indicated by wavy lines near the insertion tube 20 (the same is true hereinafter) .

Presumably, this phenomenon is explained as follows. The vibration of the insertion tube 20 lowers static pressure around the intraductal foreign body removal instrument 10. Consequently, fluid convection (eddy) toward the intraductal foreign body removal instrument 10 occurs in the duct 90. The vibration and convection produce a force which moves the fibrous foreign bodies 80 toward the free end side 10a of the intraductal foreign body removal instrument 10.
Then, as shown in Figure 2D, the fibrous foreign bodies 80 drawn to the free end side 10a get tangled around it. After the fibrous foreign bodies 80 are tangled, the intraductal foreign body removal instrument 10 is retrieved from the duct 90 through insertion position.

In so doing, as shown in Figure 2D, the insertion tube 20 tangled with the fibrous foreign bodies 80 and the wire 30 in the insertion tube 20 are stored once in the guide tube 25 together with the fibrous foreignbodies 80 before the entire intraductal foreign body removal instrument 10 is retrieved. This prevents the fibrous foreign bodies 80 from falling off the free end side as well as prevents them from damaging inner walls of the duct 90 when the intraductal foreign body removal instrument 10 is retrieved. Incidentally, in the figure, the retrieval of the free end side 10a of the intraductal foreign body removal instrument 10 from the duct 90 is conceptually indicated by an arrow near the free end side 10a. In this way, the fibrous foreign bodies 80 can be removed from the duct 90.

Thus, in the operation of removing the fibrous foreign bodies 80 out of the intravital duct 90, a catheter (intraductal foreign body removal instrument) is fed into the duct 90. With the intraductal foreign body removal instrument 10 according to the present invention, the insertion tube 20 and the wire 30 in the insertion tube 20 have flexibility. Besides, the insertion tube 20 and the wire 30 in it can have small diameters. That is, the free end side 10a consists of only the insertion tube 20 (and the wire 30 in it) and does not have a special mechanism such as a gripping mechanism. This results in low resistance in the duct 90. Also, even elbows and similar bends do not offer high resistance. This in turn increases feed limits of the intraductal foreign body removal instrument 10 into the duct 90. Also, even if the duct 90 has a small diameter, the intraductal foreign body removal instrument 10 can be inserted easily into it. Thus, patient invasion can be reduced.

After the fibrous foreign bodies 80 are tangled around the free end side 10a by the rotation of the wire 30 with the rotating device 40 as described above, they can be removed as the intraductal foreign body removal instrument 10 is retrieved from the duct 90 through the insertion position. Thus, even under circumstances where the duct 90 is narrow and tortuous allowing a limited work area, the intraductal foreign body removal instrument 10 can be suitably used as a catheter to remove the fibrous foreign bodies 80 in the duct 90.

Furthermore, the insertion tube 20 is loosely inserted into the guide tube 25. That is, the guide tube 25 and insertion tube 20 can move relative to each other in the longitudinal direction. Also, the insertion tube 20 is inserted into the guide tube 25 with a predetermined clearance which can accommodate fibrous foreign bodies 80 tangled around the free end side 10a. Consequently, the insertion tube 20 and the wire 30 in the insertion tube can be stored once in the guide tube 25 together with the foreign bodies 80 so that the free end side 10a is retrieved and foreign bodies are removed from the duct 90 through the insertion position. This suitably prevents the fibrous foreign bodies 80 from falling off the free end side as well as prevents them from damaging inner walls of the duct 90 during retrieval. Also, where the insertion tube 20 is covered by the guide tube 25, the vibration of the insertion tube 20 can be reduced by the guide tube 25.

Thus, the intraductal foreign body removal instrument 10 can remove fibrous foreign bodies 80 (occluding objects such as thrombus) clogging constrictions in the duct 90, without damaging inner walls of the duct 90 using a simple mechanism which does not require a large, complex equipment configuration.
Incidentally, the above-described intraductal foreign body removal instrument according to the present invention is not limited to the above embodiment and can be modified as required without departing from the spirit and scope of the present invention.
For example, although in the above embodiment, the intraductal foreign body removal instrument according to the present invention is used as a catheter, the present invention is not limited to this and can also be used for other applications as long as it is used as an intraductal foreign body removal instrument to remove fibrous foreign bodies in ducts. For example, it canbe used to remove hairs (fibrous foreignbodies) clogging a drain pipe.

Also, although in the above embodiment, the insertion tube 20 with the wire 30 in it is inserted into the guide tube 25, this is not restrictive and it is not strictly necessary to use the guide tube 25. However, preferably the intraductal foreign body removal instrument is equipped with a guide tube to reduce vibration by means of the guide tube 25 in locations other than near the free end side 10a where convection caused by vibration is required, prevent the fibrous foreign bodies to be removed from falling off the free end side, and prevent the fibrous foreign bodies from damaging inner walls of the duct when they are retrieved.

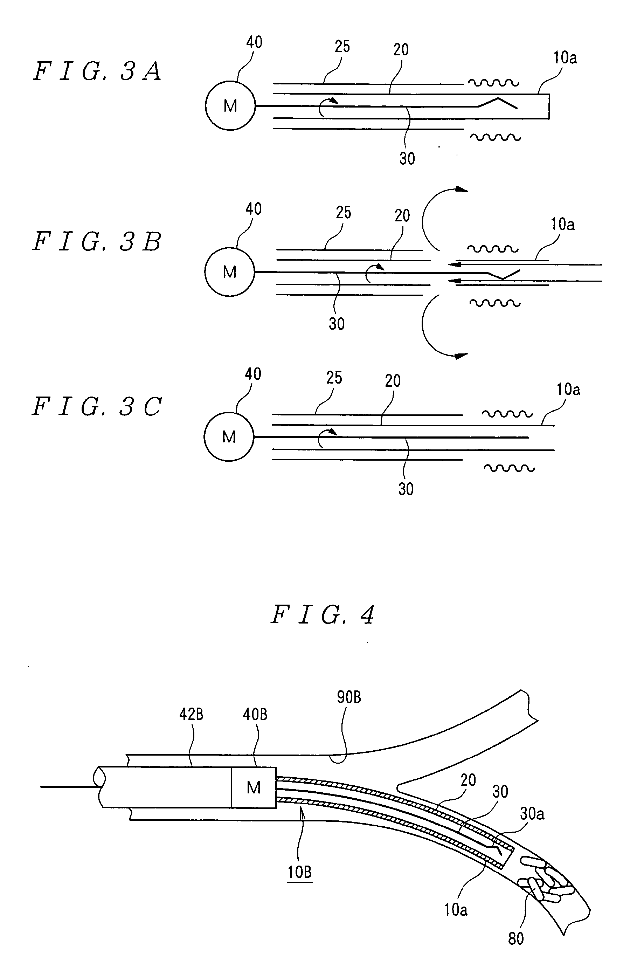

Also, although in the above embodiment, fluororesin (PFA) is used for the insertion tube 20 and guide tube 25, this is not restrictive and various materials are applicable as long as they are flexible. Also, although wire material of a nickel-titanium alloy is used for the wire 30 in the above embodiment, this is not restrictive and various materials are applicable as long as they are flexible.
Also, the shapes of the insertion tube 20 and the wire 30 are not limited to those used in the above embodiment. For example, variations of the shapes of the insertion tube 20 and the wire 30 are shown in Figures 3A to 3C.

For example, although in the above embodiment, the insertion tube 20 has a tubular shape with both longitudinal ends open, this is not restrictive and it may have a tubular shape with the free end side 10a closed as exemplified by a variation shown in Figure 3A. However, to produce convection to the free end side 10a efficiently, it is desirable to open up the free end side 10a of the insertion tube 20. Incidentally, to produce such convection more efficiently, one or more holes may be made an appropriate distance away from the end on the free end side 10a of the insertion tube 20, thereby securing flow paths, as exemplified by a variation shown in Figure 3B. Incidentally, in Figure 3B, formation of the flow paths is conceptually indicated by arrows.

Also, although in the above embodiment, for example, the free end side 10a of the wire 30 is bent in a "dogleg shape" to form the bend 30a, this is not restrictive and the free end side 10a may be straight as exemplified by a variation shown in Figure 3C instead of being bent. However, to produce convection to the free end side 10a efficiently, it is desirable to form a bend on the free end side 10a of the wire 30 as in the case of the above embodiment. Incidentally, the bend is not limited to a dogleg shape and various shapes may be used.

Also, although in the above embodiment, the wire 30 is made of a single filament of wire material, this is not restrictive and any wire will serve its purpose for the present invention as long as it can be rotated in the insertion tube. For example, the intraductal foreign body removal instrument may employ a wire consisting of twisted filaments, or multiple untwisted filaments passed through the insertion tube 20 as the wire 30.
Also, although in the above embodiment, the 0.5-mm diameter (outside diameter) insertion tube 20 and the 0.2-mm diameter wire 30 are used to reduce the diameter of the intraductal foreign body removal instrument 10, this is not restrictive and appropriate thickness may be selected depending on applications of the present invention.

Also, although in the above embodiment of the intraductal foreign body removal instrument according to the present invention, the rotating device 40 is placed outside the target duct and the insertion tube (together with the wire in it) is inserted into the duct, the present invention is not limited to this.
For example, as exemplified by a variation shown in Figure 4, a microactuator insertable into a duct 90B in which an intraductal foreign body removal instrument 10B is used can be adopted as a rotating device 40B of the intraductal foreign body removal instrument 10B according to the present invention. One end of a flexible insertion tube 42B for pull-up is attached to the rotating device 40B. The other end of the flexible insertion tube 42B for pull-up is attached to a flexible-insertion-tube winder (not shown). The flexible insertion tube 42B is hollow and wiring or piping for use to drive the rotating device 40B is connected to the rotating device 40B, passing through the flexible insertion tube 42B.

According to this variation, the intraductal foreign body removal instrument 10B itself is inserted into the duct 90B until it gets close to a constriction constricted by fibrous foreign bodies 80 in the duct 90B. Then, the free end side 10a of the intraductal foreign body removal instrument 10B is brought close to the fibrous foreign bodies 80, which then get tangled around the insertion tube 20 as the wire 30 is rotated by the rotating device 40B. Then, as the flexible insertion tube 42B is wound by the flexible-insertion-tube winder, the intraductal foreign body removal instrument 10B is retrieved from the duct 90B to remove the fibrous foreign bodies 80. Incidentally, although no guide tube 25 is used in this variation, the intraductal foreign body removal instrument 10B can be configured with a guide tube 25.

The configuration in Figure 4 is applicable, for example, when a duct has a relatively large opening and a large distance to a constriction with the bore of the duct getting narrower halfway. In such a case, if the free end side of the intraductal foreign body removal instrument is too long, the insertion tube and wire may get buckled and folded halfway through the duct, but the configuration in Figure 4 accommodates such a situation by making the free end side shorter.

In the intraductal foreign body removal instrument 10 according to the present invention, although the axial lengths of the insertion tube 20 and wire 30 are shown in Figure 1 as being almost equal and the free end side 10a of the guide tube 25 is shown as being shorter than the insertion tube 20, this is not restrictive. These tubes and the like may have different lengths at least in the target duct as long as they serve their purpose for the present invention. For example, the wire 30 alone may be increased in length on the side where the wire 30 is connected to the rotating device 40 while the guide tube 25 and insertion tube 20 have the same length. Also, the intraductal foreign body removal instrument according to the present invention can have a large overall length because it allows a large amount of feed when feeding the free end side into the duct, but it can also be used for short-range applications.

### (Example)

Next, description will be given of an example (experiment) in which the intraductal foreign body removal instrument according to the present invention is used.
This example is a basic experiment conducted to check operation and effect of the intraductal foreign body removal instrument with the configuration of the above embodiment when it is used as a catheter inserted into an intravital duct. Incidentally, the intraductal foreign body removal instrument used in this experiment was the same as the intraductal foreign body removal instrument 10 described above except that it was not equipped with the guide tube 25, and thus detailed description thereof will be omitted.

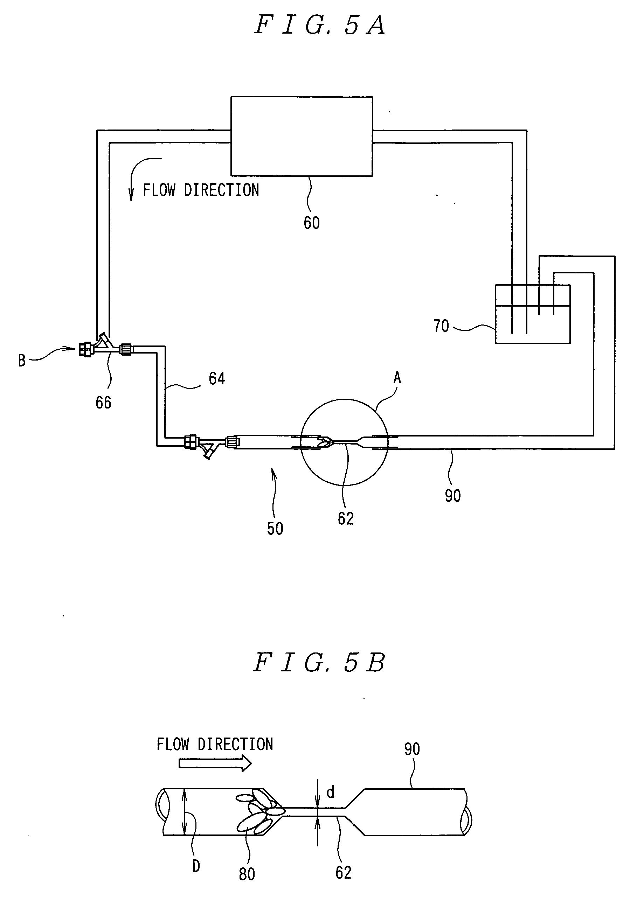

Figure 5A is a schematic block diagram illustrating an overall experimental arrangement.
As shown in the figure, a blood vessel model 50 was constructed of a glass tube to represent an intravital duct 90. The blood vessel model 50 could pump up water used to represent blood from a tank 70 and circulate it through the duct 90 using a circulation unit 60 equipped with a pump.

Specifically, as illustrated by the enlarged view of part A shown in Figure 5B, a narrowed part 62 was provided to represent constriction caused by a thrombus by reducing the part of the glass tube in diameter. To produce the narrowed part 62, the center of a glass tube with an inside diameter D of 5.8 mm had its outside diameter d reduced to approximately 1 mm by heating with a burner. Then, a thrombus (fibrous) formed of blood sampled from a rabbit was plugged into one end of the narrowed part 62 as fibrous foreign bodies 80.
As shown in Figure 5A, an elbow 64 was constituted of a pipe with multiple bends (two bends in the figure) and connected to one end of the narrowed part 62 to represent a blood vessel in a living body. A Y-shaped connecting pipe 66 was connected to the other end of the elbow 64, allowing a catheter (the intraductal foreign body removal instrument according to the above embodiment) to be inserted into one branch of the Y-shaped pipe (as indicated by arrow B in the figure).

An experiment to remove the fibrous foreign bodies 80 clogging the narrowed part 62 was conducted using the intraductal foreign body removal instrument 10 on the blood vessel model 50 configured as described above.
First, the catheter (the intraductal foreign body removal instrument 10) was inserted in one branch of the Y-shaped connecting pipe 66 and passed through the multiple bends in the elbow 64. Since the 0.5-mm diameter (outside diameter) insertion tube 20 and 0.2-mm diameter wire 30 were used to reduce the diameter of the catheter (the intraductal foreign body removal instrument 10) as described in the above embodiment, the catheter could be fed easily to near one end of the narrowed part 62 (at the left of the foreign bodies 80 in Figure 5B).

Next, the wire 30 was rotated by the rotating device 40. As a result, it was observed that the foreign bodies 80 were drawn to the free end side 10a of the intraductal foreign body removal instrument 10 and tangled around it as described in the above embodiment. The rotational speed of the rotating device 40 was ranged between 8,000 rpm and 18,000 rpm and an effect was observed in the entire range of rotational speed. It was found that as the rotational speed was increased, stronger convection (eddy) was produced, drawing the foreign bodies 80 more strongly to the free end side 10a.
It was confirmed that after being tangled around the free end side 10a, the foreign bodies 80 could be removed by withdrawing the intraductal foreign body removal instrument 10 from the connecting pipe 66 serving as insertion position into the duct 90.
Thus, it was confirmed that the intraductal foreign body removal instrument 10 according to the present invention could remove fibrous foreign bodies 80 out of the duct 90 without damaging inner walls of the duct 90 using a simple mechanism.

### Industrial Applicability

The intraductal foreign body removal instrument according to the present invention can remove fibrous foreign bodies out of a duct without damaging inner walls of the duct using a simple mechanism.

## Claims

1. An intraductal foreign body removal instrument which removes fibrous foreign bodies out of a duct, **characterized by** comprising:
a flexible insertion tube inserted in the duct; a wire made of flexible wire material and inserted in the insertion tube; and a rotating device which rotates the wire in the insertion tube.

2. The intraductal foreign body removal instrument according to claim 1, **characterized in that** the intraductal foreign body removal instrument is a catheter inserted into an intravital duct.

3. The intraductal foreign body removal instrument according to claim 1 or 2, **characterized by** further comprising a flexible guide tube which is inserted in the duct and into which the insertion tube is inserted loosely.
